# EUROPEAN PATENT APPLICATION

(11) **EP 1 909 105 A1**
(43) Date of publication of application: **09.04.2008**
(21) Application number: 06394020.9
(22) Date of filing: 06.10.2006
(51) Int. Cl.: G01N 33/86

(54) **A method of determining platelet function**

(71) Applicant: Royal College of Surgeons in Ireland, Dublin 2 (IE)
(72) Inventor: The designation of the inventor has not yet been filed
(74) Representative: Purdy, Hugh Barry

(57) **Abstract**

According to the invention, there is provided a method of analysing platelet function in an individual comprising the steps of providing a platelet-containing biological sample, providing at least three platelet function modulators, each platelet function modifier being provided in a range of concentrations, reacting aliquots of the platelet containing sample with each concentration of each platelet function modulator in a separate reaction vessel, measuring the degree of aggregation of platelets in each reaction vessel, and determining the platelet function profile of the platelets to each of the platelet function modifiers.

## Description

### Introduction

The invention relates to a method of determining platelet function, and assay kits suitable for carrying out the method.

Cardiovascular disease remains the leading cause of mortality in Europe and the USA. The conundrum remains as to who will suffer from either a heart attack or stroke. Multiple risk prediction models have been formulated to try to succeed in predicting the high risk patients in the population, with some success. Cardiovascular events occur as a result of thrombosis. Thrombosis is the act of clot formation and occurs as a result of platelet activation. This is prevented in part by aspirin, however events still occur. This suggests that the "stickiness" or level of activation between individuals differs and the response to therapy differs also. It is surprising that to this day no one has a certain idea as to what constitutes normal platelet function. This is largely due to the limitations in working with platelets. Unfortunately platelets only survive for approximately 4-6 hours after leaving the body. The process of preparing ex vivo platelets shortens this duration and so only a small number of tests can be performed by an individual, hence providing only a small amount of information on platelet function.

There a small number of commercially available platelet function analysers on the market which have some limitations. The PFA (platelet function analyzer)-100 device measures time to clotting after exposing whole blood to collagen and epinephrine or collagen and ADP known as closure time. The parameter being measured is the closure time. The gold standard test for platelet function is Light Transmission Standard Aggregometry. When one compares PFA-100™ to the gold standard platelet function test the results differ highlighting a major limitation of the device. Unfortunately the Standard aggregometer is limited in that the procedure takes a considerable amount of time to even do a small number of channels as the device tends to have only 4 channels. Accumetrics Verify NOW™ device is another device which is used at the bedside, using whole blood to assess platelet function. This device allows one to assess response to Aspirin and Clopidogrel. This test like the PFA-100™ device is limited in that it provides only a limited amount of information regarding platelet reactivity. Further, all of these tests only examine the effects of agonist at a single concentration.

The assessment of platelet reactivity or the ability of the platelet to activate differs between individuals and varies within the same individual at varying time points. Assessing this variability using the currently available tests is difficult, time inefficient and expensive.

It is an object of the invention to overcome at least one of the above problems.

### Statements of Invention

According to the invention, there is provided a method of analysing platelet function in an individual comprising the steps of
- providing a platelet-containing biological sample;
- providing at least three platelet function modulators, each platelet function modifier being provided in a range of concentrations;
- reacting aliquots of the platelet containing sample with each concentration of each platelet function modulator in a separate reaction vessel;
- measuring the degree of aggregation of platelets in each reaction vessel; and
- determining the platelet function profile of the platelets to each of the platelet function modifiers.

Thus, the method essential provides a means for analysing platelet function in a high throughput manner which employs the use of a microtitre plate (or an equivalent having a multiplicity of reaction wells) and a range of platelet function modulators (i.e. agonists, antagonists, drugs etc) at a range of concentrations, and measuring platelet aggregation in each of the wells at a range of time points from time 0, and optionally preparing dose response curves for each platelet function modulator.

Typically, at least four platelet function modulators are employed. Preferably, at least five platelet function modulators are employed. Ideally, more than five platelet function modulators are employed.

Suitably, the platelet function modulators are either platelet agonists or platelet antagonists. Ideally, the platelet function modulators are platelet agonists.

In one embodiment, the platelet function modulators are selected from the group comprising: TRAP; collagen; epinephrine; ADP; arachidonic acid; serotonin; and thromboxane A2; ristocetin; a NO donor (such as SNAP); U46619; and Convulxin. Typically, the platelet function modulators are selected from the group comprising: TRAP; collagen; epinephrine; ADP; and arachidonic acid.

When TRAP is employed, it is preferable to add TRAP to the microtitre plate after all the other platelet function modulators have been added. Ideally, the TRAP is added to the plate immediately prior to addition of the platelet-containing sample.

Suitable platelet antagonists will be well known to a person skilled in the field of platelet biology.

Typically, the multiple reactions are carried out in a microtitre plate, and the reaction vessels are the wells of the microtitre plate. Ideally, a 96 well mirotitre plate is employed. Alternatively, the multiple reactions may be carried out in a miniaturised version of the microtitre plate.

In this specification, the term "range of concentrations" as applied to platelet function modulators means that the modulator is provided in at least three, four, five, six, seven, eight, or nine concentrations. Typically, arachidonic acid will be provided at concentrations in the range of 1 to 1000µM, preferably in the range of 3 to 700µM, and more preferably in the range of 5 to 500µM. Typically, collagen will be provided in the range of 0.001 to 0.3mg/ml, preferably in the range of 0.0015 to 0.25mg/ml, and more preferably in the range of 0.0023 to 0.19mg/ml. Typically, ADP will be provided in the range of 0.01 to 40 µM, preferably 0.2 to 30 µM, and more preferably 0.25 to 20µM... Typically, epinephrine will be provided in the range of 0.01 to 40µM, preferably 0.125 to 30µM, and more preferably 0.15 to 20µM. Typically, TRAP will be provided in the range of 0.01 to 40 µM. Generally, the range of concentration employed for the platelet function modulators should span four log units, and should encompass the known effective dose range for each of the platelet function modulators employed.

In this specification, the term "platelet function" refers to the activation status of the platelet sample, i.e. the ability of the platelet to aggregate with other platelets. Suitably, aggregation of platelets is determined by light transmission aggregometry. In this method, light passing through the well containing the reaction mixture is measured prior to and after addition of the platelet function modulator. Thus, when a platelet agonist is added, the absorbance of light passing through the reaction mixture will generally increase due to aggregation of the activated platelets in the well. Generally, light absorbance is measured using a wavelength of between 550 and 590nm, preferably between 560 and 580nmm and more preferably between 570 and 575nm. Ideally, light absorbance is measured using a wavelength of 572nm. Light transmission readers suitable for taking light readings from multiple plates will be well known to those skilled in the art. Further, light absorbance may be measured at different wavelengths such as, for example, 405nm, 490nm, and other wavelengths.

The platelet-containing sample is generally selected form the group comprising: washed platelet preparations; and platelet rich plasma (PRP). Suitably, the concentration of platelets in the platelet-containing sample is greater than 2 x 10⁵/µl, typically greater than 3 x 10⁵/µl, preferably greater than 4 x 10⁵/µl, and ideally greater than 5 x 10⁵/µl. Ideally, the PRP is obtainable by aspirating blood into a solution of citrate, typically a solution of sodium citrate, and generally in a ratio of 9:1 blood:citrate. Typically, the citrate solution has a concentration of between 3% and 4%, (w/v). Typically, the volume of agonists/platelet-containing sample (including buffer) added to each well is between 150 and 250 µl, preferably between 180 and 220 µl, most preferably between 195 and 205 µl, and ideally about 200 µl.

Typically, the level of aggregation in each reaction vessel is measured at different time points following the reaction of the platelets with the platelet function modulator. Suitably, at least 2, 3, 4, 5, 6, 7, 8, 9, 10, 12, or 15 readings will be taken during the first 30 minutes, preferably during the first 25 minutes, and most preferably during the first 20 minutes of the reaction. Suitably, readings are taken every 2 to 5 minutes. Ideally, readings are taken at 0, 3, 9, 15, and 18 minutes. Ideally, the plate is agitated between readings, typically employing an orbital shaker. Ideally, the orbital shaker operates at an orbital shaking pattern of between 0.5 and 3mm, preferably about 1mm.

The term "platelet function profile" refers to the activation status of the platelet sample in response to different platelet function modulators; such a profile will give an indication at any time point of the actual activation status of a platelet sample to, for example, a number of different agonists. Typically, determining a platelet function profile for an individuals platelets comprises preparing a dose response curve for each of the platelet agonists employed. Generally, the platelet function profile is determined by calculating, for each of the platelet agonists, a characteristic of the dose response curve selected from the group comprising: EC₅₀ value (the effective concentration at which 50% aggregation of platelets occurs); hill slope; and minimum and maximum readings.

In another embodiment, the invention relates to a method of assisting in determining a clinical status of an individual comprising the steps of determining the platelet function profile of the individuals platelets according to the method of the invention, and correlating the reactivity profile thus obtained with a reference platelet function profile which is indicative of a clinical status, whereby correlation of a measured platelet function profile with a reference profile in the library provides assistance in determining a clinical status of the individual. Suitably, the reactivity profile obtained is correlated with a library of platelet function profiles, each platelet function profile in the library being suitably indicative of a clinical status. The library may contain, for example, platelet function profiles that are indicative of a clinical status selected form the group comprising: normal healthy male and females; drug responsiveness; drug resistanace; surgical risk; cardiovascular risk status; platelet transfusion risk. Drug responsiveness clinical status may include responsiveness to anti-thrombosis agents, such as those selected from the group comprising: aspirin-related drugs; ADP-receptor inhibiting drugs; and GPIIbIIIa antagonists/blockers. Thus, the methods of the invention may be used to quickly determine the most suitable anti-thrombosis therapy for an individual, given their platelet function profile. Further, the methods may be used to determine whether an individual is at risk of bleeding during surgery. In one embodiment, the reference platelet function profile may be a profile of a normal healthy male or female donor, in which case correlation will indicate whether the platelet function profile of the patenits sample is indicative of normal healthy platelet function. However, if for example the EC₅₀ values obtained from the patient sample are significantly different (i.e. lower) from those of the normal sex-matched reference, then this could indicate that the patients platelets are hyper-responsive thereby predisposing the patient thrombotic events. In this regard, the methods of the invention provide a method of prognosis of cardiovascular morbidity and/or mortality.

The invention also relates to a kit suitable for rapid, high throughput, assaying of platelet function, the kit comprising:
- a mirotitre plate;
- at least three platelet function modulators; and
- instructions for carrying out a method of the invention.

In another embodiment, the invention relates to a kit suitable for rapid, high throughput, assaying of platelet function, the kit comprising a mirotitre plate, the wells of the microtitre plate containing a plurality of concentrations of at least three platelet function modulators, (i.e. platelet agonists or platelet antagonists), each concentration of each platelet function modulator being contained within separate wells of the plate. Typically, the platelet function modulators are stored in the microtitre plate for at least 1, 2, 3, 4, 5, 6 or 7 days. However, when TRAP is employed, it is preferable to load the TRAP onto the plate immediately in advance of the assay. In one embodiment, the kit is packaged with the platelet function modulators in-situ in the wells of the plate.

Preferably, the microtitre plate holds a range of concentrations of at least four platelet function modulators, and ideally at least five platelet function modulators.

Typically, the kit comprises at least 4, and preferably at least five, platelet function modulators. Ideally, the platelet function modulators are platelet agonists. Suitably, the plate holds at least two, three, four, five, six, seven, or eight different concentrations of each platelet function modulator. Typically, the platelet function modulators are in liquid form. However, in an alternative embodiment, the platelet function modulators are in a lyophilised form (in which case the platelet function modulators may be stored for weeks or months, optionally stored in the wells of the plate for weeks or months). This is achieved, for example, by adding a solution of the various modulators (i.e. platelet agonists) to the wells of the plate, and then placing the plate in a freeze dryer to lyophilise the agonist solutions, leaving just lyophilised agonist in the wells of the plate. The plates may then be covered by a suitable cover, such as for example, a peel-off top, or capping lids of the type sold for use with microtitre plates.

Thus, in one embodiment, the platelet agonists are lyophilised in-situ in the wells of the microtitre plate.

In one embodiment, the platelet function modifiers are selected from the group comprising: TRAP; collagen; epinephrine; ADP; arachidonic acid; serotonin; thromboxane A2; convulxin; a NO donor (i.e. SNAP); and U46619. Typicaly, the platelet function modifiers are selected from the group comprising: TRAP; collagen; epinephrine; ADP; and arachidonic acid.

Ideally, the microtitre plate is a 96 well mirotitre plate. As an alternative, a miniaturised, or modified, version of the microtitre plate may be employed.

Generally, the instructions indicate that the platelet function modifier is added to the plate in at least three, four, five, six, seven, eight, or nine concentrations. Typically, arachidonic acid will be provided at concentrations in the range of 1 to 1000µM, preferably in the range of 3 to 700µM, and more preferably in the range of 5 to 500µM. Typically, collagen will be provided in the range of 0.001 to 0.3mg/ml, preferably in the range of 0.0015 to 0.25mg/ml, and more preferably in the range of 0.0023 to 0.19mg/ml. Typically, ADP will be provided in the range of 0.01 to 40 µM, preferably 0.2 to 30 µM, and more preferably 0.25 to 20µM.. Typically, epinephrine will be provided in the range of 0.01 to 40µM, preferably 0.125 to 30µM, and more preferably 0.15 to 20µM.. Typically, TRAP will be provided in the range of 0.01 to 40 µM, preferably 0.2 to 30 µM, and more preferably 0.25 to 20µM.

In one embodiment, the invention provides a kit suitable for determining a clinical status of an individual according to a method of the invention, the kit comprising a kit according to the invention and a library of platelet reactivity profiles, each profile being indicative of a clinical status, including normal healthy clinical status. Typically, the library of profiles are provided in an electronic format, optionally along with software enabling a user correlate a measured profile with the library of profiles. Suitably, the library comprises at least one platelet reactivity profile. Suitably, the clinical status is selected from the group comprising: normal healthy male or females; drug responsiveness; drug resistanace; surgical risk; and cardiovascular risk status. Drug responsiveness clinical status may include responsiveness to anti-thrombosis agents, such as those selected from the group comprising: aspirin-related drugs; ADP-receptor inhibiting drugs; and GPIIbIIIa antagonists/blockers. Thus, the methods of the invention may be used to quickly determine the most suitable anti-thrombosis therapy for an individual, given their platelet function profile. Further, the methods may be used to determine whether an individual will be at risk of bleeding during surgery. Further, the methods and kits of the invention may be employed to make a prognosis of future cardiovascular and thrombotic events, and as such could be used to as a means of prognosis of cardiovascular morbidity or mortality.

Ideally, the kit includes instructions for carrying out a method of the invention.

### Detailed Description of the Invention

The invention will be more clearly understood from the following description of an embodiment thereof, given by way of example only with reference to the accompanying Figures in which:

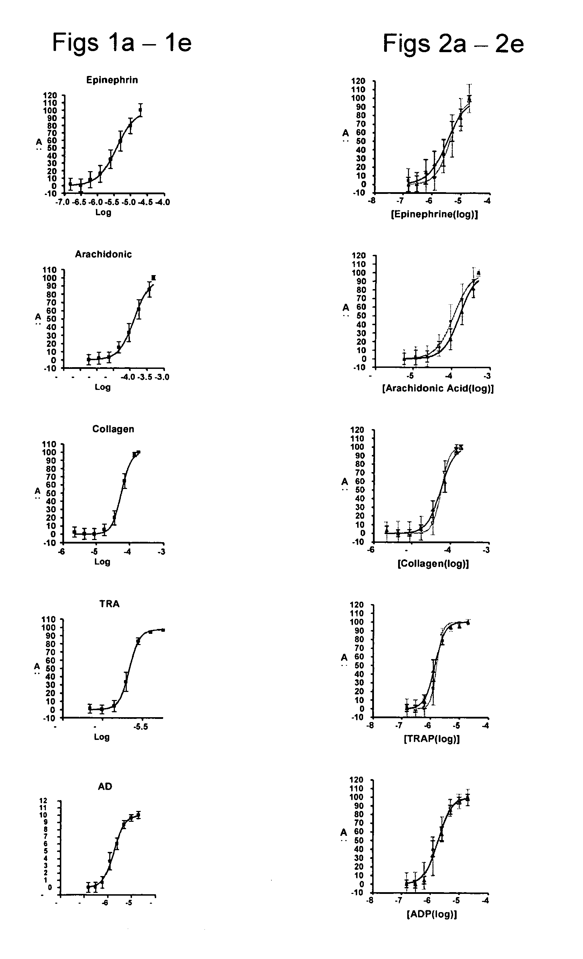

Figs 1a to 1e are dose response curves from 14 healthy donors for each of five agonists; and
Figs 2a to 2e are dose response curves comparing 7 men (dark line) and 7 women (light line) for each of five agonists.

### METHOD

### Platelet Preparation

Blood is aspirated into a 60ml syringe containing 6 ml of 3.8% sodium citrate. After gentle mixing 5ml of this blood is aliquoted into 15ml Falcon tubes and then centrifuged once at 150G for 10 min. Following this, the top layer known as Platelet Rich Plasma (PRP) is removed from each of the 12x5ml aliquots.

### Microtitre Plate Preparation

Using a 96 well black isoplate with clear bottom wells 5 different agonists are pipetted into each row. The agonists used are Arachidonic Acid, Collagen, Adenosine Diphosphate (ADP), Epinephrine and Thrombin Related Activated Peptide (TRAP). The starting well is filled with the stock concentrations and sequentially diluted to give 8 concentrations of each agonist ranging as follows:
- Arachidonic Acid: (5.86-500µM)
- Collagen: (0.0023-0.19mg/ml)
- ADP: (0.01 - 40µM)
- Epinephrine: (0.01 - 40µM)
- TRAP: (0.01 - 40µM)

These plates are then refrigerated at 2-8°C overnight in a covered 96 well plate, to be used the following day. The prepared plates are taken out of the fridge for a minimum of 30minutes prior to use.

### Rapid High-Throughput Assay

The PRP is transferred using a multichannel pipette to a 96 well plate which has been pre-prepared the previous day as described avove. This is then placed in a Wallac Victor 2 plate reader and read at different time points. The plate is read at time 0, 3, 9, 15, 18min and records light absorbance at 572nm. In between these time points the plate is rotated using an orbital shaking pattern at 1mm diameter. The data generated is analysed using PRISM software to generate dose response curves for each of the agonists. From this the EC₅₀ (effective concentration at which 50% aggregation occurs) is generated and can then be compared for each of the platelet agonists.

The present assay generates a mass of information with regard to platelet biology which cannot be achieved using any of the other platelet function assay currently available. In conjunction with the PRISM software it generates actual figures such as EC50 and hill-slope which cannot be done with the other tests mentioned. Even when compared to the gold standard of light transmission Aggregometry this high through put method actually generates real meaningful data that can be analysed easily as well as providing at least 20 times the data generated from standard Aggregometry. This high through put technique has previously been validated against standard aggregation successfully which has not been seen consistently with other platelet function analysers.

In conclusion this assay provides more extensive information on platelet biology than that seen with other similar assays as well as providing much needed information on platelet reactivity. It provides the same information regarding response of platelets to therapies such as Aspirin and Clopidogrel. It allows one to examine platelet reactivity and compare this using EC50 at multiple time points and in response to any therapy.

The invention is not limited to the embodiment hereinbefore described, but may be varied in construction and detail without departing from the spirit of the invention.

## Claims

1. A method of analysing platelet function in an individual comprising the steps of
- providing a platelet-containing biological sample;
- providing at least three platelet function modulators, each platelet function modulator being provided in at least two concentrations;
- reacting aliquots of the platelet containing sample with each concentration of each platelet function modulator in a separate reaction vessel;
- measuring the degree of aggregation of platelets in each reaction vessel; and
- determining the platelet function profile of the platelets to each of the platelet function modifiers.

2. A method as claimed in Claim 1 in which the platelet function modulators are platelet agonists.

3. A method as claimed in Claim 1 or 2 in which at least four platelet function modulators are employed.

4. A method as claimed in Claim 1, 2 or 3 in which at least five platelet function modulators are employed.

5. A method as claimed in any of Claims 2 to 4 in which the platelet function modulators are selected from the group comprising: TRAP; collagen; epinephrine; ADP; arachidonic acid; NO donors; convulxin; and U46619.

6. A method as claimed in any preceding Claim in which the multiple reactions are carried out in a microtitre plate, and the reaction vessels are the wells of the microtitre plate.

7. A method as claimed in any preceding Claim in which each platelet function modulator is provided in at least three concentrations.

8. A method as claimed in any preceding Claim in which each platelet function modulator is provided in at least six concentrations.

9. A method as claimed in any preceding Claim in which each platelet function modulator is provided in at least eight concentrations.

10. A method as claimed in any preceding Claim in which the aggregation of platelets is determined by light transmission aggregometry.

11. A method as claimed in any preceding Claim in which the platelet-containing sample is selected form the group comprising: washed platelet preparations; and platelet rich plasma.

12. A method as claimed in any preceding Claim in which the degree of aggregation in each reaction vessel is measured at different time points.

13. A method as claimed in any of Claims 2 to 12 comprising the step of determining the reactivity profile of the individuals platelets for each of the platelet agonists.

14. A method as claimed in Claim 13 in which a dose response curve is generated for each of the platelet agonists

15. A method as claimed in Claim 13 in which the reactivity profile is determined by calculating, for each of the platelet agonists, a characteristic of the dose response curve selected from the group comprising: EC₅₀ value; hill slope; minimum readings; and maximum readings.

16. A method of determining a clinical status of an individual comprising the steps of determining the reactivity profile of an individuals platelets according to a method of Claim 14 or 15, and comparing the reactivity profile with a library of reactivity profiles, each reactivity profile in the library being indicative of a clinical status.

17. A method as claimed in Claim 16 in which the clinical status is selected form the group comprising: normal and healthy; drug responsiveness; drug resistanace; bleed risk; and cardiovascular risk status.

18. A kit suitable for rapid, high throughput, assaying of platelet function, the kit comprising:
- a mirotitre plate;
- at least three platelet function modulators; and
- instructions for carrying out a method of any of Claims 1 to 12.

19. A kit as claimed in Claim 18 in which the platelet function modulators are platelet agonists.

20. A kit as claimed in Claim 19 comprising at least four platelet agonists.

21. A kit suitable for rapid, high throughput, assaying of platelet function, the kit comprising a mirotitre plate, the wells of the microtitre plate having a plurality of concentrations of at least three platelet agonists.

22. A kit as claimed in Claim 21 in which the microtitre plate holds a plurality of concentrations of at least four platelet agonists.

23. A kit as claimed in Claim 22 in which the microtitre plate holds a plurality of concentrations of at least five platelet agonists.

24. A kit as claimed in any of Claims 21 to 23 in which the plate holds at least three different concentrations of each agonist.

25. A kit as claimed in any of Claims 21 to 23 in which the plate holds at least five different concentrations of each agonist.

26. A kit as claimed in any of Claims 21 to 23 in which the plate holds at least eight different concentrations of each agonist.

27. A kit as claimed in any of Claims 21 to 26 in which the platelet agonists are in liquid form.

28. A kit as claimed in any of Claims 2 to 26 in which the platelet agonists are in a lyophilised form.

29. A kit as claimed in Claim 28 in which the platelet agonists are lyophilised in-situ in the wells of the microtitre plate.

30. A kit as claimed in any of Claims 21 to 29 and including instructions for carrying out a method according to any of Claims 1 to 12.

31. A kit suitable for determining a clinical status of an individual according to a method of Claim 16, the kit comprising a kit according to any of Claims 18 to 30, and a library of platelet reactivity profiles.

32. A kit as claimed in Claim 31 in which the clinical status is selected from the group comprising: normal and healthy; drug responsiveness; drug resistance; surgical risk; and cardiovascular risk status.
